(19) Europäisches Patentamt / European Patent Office / Office européen des brevets

(11) **EP 3 015 120 B1**

(12) **EUROPEAN PATENT SPECIFICATION**

(45) Date of publication and mention
of the grant of the patent:
**07.08.2019 Bulletin 2019/32**

(21) Application number: **14818651.3**

(22) Date of filing: **26.06.2014**

(51) Int Cl.:
*A61L 27/40* *(2006.01)*      *A61L 27/14* *(2006.01)*
*A61L 27/20* *(2006.01)*      *A61L 27/22* *(2006.01)*
*A61L 27/24* *(2006.01)*      *A61L 27/54* *(2006.01)*
*A61L 27/56* *(2006.01)*      *A61L 27/60* *(2006.01)*
*A61L 31/12* *(2006.01)*      *A61L 31/14* *(2006.01)*
*A61L 31/16* *(2006.01)*      *A61L 27/44* *(2006.01)*

(86) International application number:
**PCT/CN2014/080798**

(87) International publication number:
**WO 2014/206308 (31.12.2014 Gazette 2014/53)**

(54) **TISSUE REPAIR SCAFFOLD AND PREPARATION METHOD AND PURPOSE THEREOF**

GEWEBEREPARATURGERÜST UND HERSTELLUNGSVERFAHREN UND ZWECK DAVON

SUPPORT DE RÉPARATION DE TISSU, SON PROCÉDÉ DE PRÉPARATION ET SA RAISON

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.06.2013 CN 201310269863
30.05.2014 CN 201410238853**

(43) Date of publication of application:
**04.05.2016 Bulletin 2016/18**

(73) Proprietor: **Medprin Regenerative Medical
Technologies Co., Ltd.
Guangzhou, Guangdong 510663 (CN)**

(72) Inventors:
• **DENG, Kunxue
Guangzhou
Guangdong 510663 (CN)**
• **YANG, Yaya
Guangzhou
Guangdong 510663 (CN)**
• **YUAN, Yuyu
Guangzhou
Guangdong 510663 (CN)**

(74) Representative: **Algemeen Octrooi- en
Merkenbureau B.V.
P.O. Box 645
5600 AP Eindhoven (NL)**

(56) References cited:
CN-A- 1 919 352      CN-A- 1 961 974
CN-A- 101 156 962      CN-A- 101 703 807
CN-A- 102 277 737      CN-A- 103 127 554
CN-A- 103 147 225      US-A1- 2009 163 936

Remarks:
The file contains technical information submitted after the application was filed and not included in this specification

## Description

## Technical Field

The present disclosure relates to a medical device and preparation thereof, particularly relates to a tissue repair scaffold and preparation method and uses thereof.

## Background Art

Tissue repair scaffolds are quite common in clinical work, e.g. tendon repair patches, ligament repair patches, hernia repair patches, dura mater repair patches, female pelvic floor dysfunction repair patches; repair of blood vessels, sling for repair; repairs of skin, fistulae, nerve conduits and bone coloboma.

The existing material itself and the pore structure have some deficiencies. It has great difference from the composition units of human tissue. Particularly, since the hydrophobic synthetic material prepared by common methods lacks porous structures similar with those observed in extracellular matrix, therefore the material is incompatible with the characteristics of the tissue anatomical structure; due to the poor tissue compliance, it is difficult for the material to form favorable adhesion with the tissue anatomical structures.

Among the present fibrous membranes for tissue repair, a woven mesh is the most widely used product. However such products usually have problems as follows: 1) the product has rough surface, relatively hard texture, and poor tissue compliance. When the product is used, the poor tissue compliance leads to friction between the product and the tissue, which makes patient uncomfortable. It may produce foreign body sensation and pain, and lead to common complications such as erosion and infection; 2) due to the poor biocompatibility, such material triggers severe immunological rejection, and leads to extensive sequelae of surgery; when being in direct contact with viscera and organs, the material is prone to produce damage, and it may cause severe adhesion, triggers serious foreign body and immune responses, therefore a secondary surgery is required to take the material out, which brings patient pain and even life-threatening. An electrospinning membrane in current study often has the defect that the growth of the cells into the membrane is difficult or slow. The properties of the existing fibrous membrane for tissue repair formed by weaving or electrospinning technology are far from ideal.

An ideal tissue repair material requires: 1) relatively large porosity, to facilitate the adhesion, crawling and growth of the cells, and to achieve rapid tissue regeneration; and the material should be able to sustain the internal pressure of the human body before the healthy tissue is completely formed; 2) when implanted into the human body, the material keeps stable in dimension, i.e. shrinkage or deformation is not allowed; 3) being soft in hand feel, so that the material is easy to form; thereby the manipulation in surgery is improved, the discomfort of the patient is relieved, and the effect of the surgery is enhanced; 4) good biocompatibility, so that tissue growth can be suitably directed, and ideal repair is achieved.

## Summary of the invention

Technical problems

The existing tissue repair material has simple pore structure and limited pore size and specific surface area, therefore the fibroblasts (approximately 30 $\mu$m in size) which generate new tissue are not able to penetrate into the material, and the newly generated tissue grows slowly.

The pore structure of the existing synthetic tissue repair material lacks diversity, and greatly differs from the structure of human tissue unit. Therefore the interaction strength between the tissue repair scaffold and the tissue is weak; the material lacks compliance to the tissue anatomical structure.

The existing woven mesh product has rough surface, relatively hard texture and poor tissue compliance. When the product is used, the poor tissue compliance leads to friction between the product and the tissue, which makes patient uncomfortable. The foreign body sensation, pain, and common complications such as erosion and infection may be produced.

The existing hydrophobic material has high intrinsic mechanical strength, but the material also possesses relatively hard texture and strong hydrophobicity. It is hard for the solution to penetrate into the material immersed therein. Even combined with techniques like freeze-drying, it is still difficult to change the rigid structure and pore structure of the material to generate compliance to the tissue anatomical structure, attachment and multiple pore structures.

The existing hydrophilic material itself is rich in hydrophilic flexible groups, while lacks rigid groups. Therefore the mechanical strength of the material is poor. A majority of the hydrophilic groups readily degrade in the *in vivo* body fluid environment. Degradation that occurs before the complete repair of the newly generated tissue leads to repair failure.

Solution to the problems

The present disclosure provides a tissue repair scaffold, wherein the tissue repair scaffold comprises a surface of at least one layer of composite fiber layer(s), said composite fiber layer comprises composite fibers formed with an adhesion factor and a hydrophobic synthetic material, said composite fiber layer being formed by interweaving composite fiber filaments with diameters of 10 nm-100 $\mu$m, and the content of said adhesion factor being 5%-50% by mass based on the total weight of the composite fiber.

The tissue repair scaffold of the present disclosure, wherein said composite fiber has a hydrophilic-lipophilic balance (HLB) value of 1-13. The tissue repair scaffold of the present disclosure, wherein the contact angle $\theta$ between the surface of the tissue repair scaffold and water is 90° or less. The tissue repair scaffold of the present disclosure, wherein the water absorption ratio of the tissue repair scaffold is larger than 100%.

The present disclosure further provides a tissue repair scaffold, wherein the tissue repair scaffold comprises a porous three-dimensional (3D) reticulate structure formed with a composite fiber layer and a hydrophilic material; said composite fiber layer comprises composite fibers formed with an adhesion factor and a hydrophobic synthetic material.

The tissue repair scaffold of the present disclosure, wherein the porous 3D reticulate structure may be formed on and/or inside the surface of the composite fiber layer. Wherein, the porous 3D reticulate structure is formed through the steps of: immersing the composite fiber layer into a solution of hydrophilic material; taking out the composite fiber layer; and freeze-drying the composite fiber layer. The solution of hydrophilic material is an aqueous solution of water-soluble polymer; said hydrophilic material is selected from the group consisting of proteins, celluloses, alcohols, ethers, chitosans, saccharides, nitrogen-containing hydrophilic substances. When the tissue repair scaffold of the present disclosure is immersed in water for 10s, the water absorption ratio thereof may reach 100-5000%. The tensile strength of the tissue repair scaffold is larger than 1 Mpa; the hydrophilic-lipophilic balance (HLB) value of the tissue repair scaffold is 1-13; the contact angle $\theta$ between the surface of the tissue repair scaffold and water is 90° or less. Wherein, once the tissue repair scaffold attaches to the tissue around, the burst strength is 0.10 kPa or more. The pore size of the tissue repair scaffold is 1.3-500 $\mu$m.

The tissue repair scaffold of the present disclosure, wherein the adhesion factor which forms the composite fiber may present inside the composite fiber and/or at the surface of the composite fiber.

The tissue repair scaffold of the present disclosure, wherein the adhesion factor is one or more selected from proteins with hydrophilicity and derivative materials thereof, celluloses with hydrophilicity and derivative materials thereof, alcohols with hydrophilicity and derivative materials thereof, chitosans and derivative materials thereof, saccharides and derivative materials thereof, and nitrogen-containing hydrophilic substances.

The tissue repair scaffold of the present disclosure, wherein the hydrophobic synthetic material includes hydrophobic polyurethane, polyethylene terephthalate, polycaprolactone, polyglycolic acid, poly(lactic acid-glycolic acid) copolymers, polymers of 1,3-propylene glycol, poly(lactic acid-caprolactone) copolymers, polylactic acid.

The tissue repair scaffold of the present disclosure, wherein the content of the adhesion factor is 5%-95% by mass, preferably 10%-50% by mass, more preferably 15%-30% by mass, based on the total weight of the composite fiber.

The tissue repair scaffold of the present disclosure, wherein the composite fiber layer is formed by interweaving composite fiber filaments with diameters of 10nm-100$\mu$m, and the composite fiber layer has a porous structure.

The tissue repair scaffold of the present disclosure, wherein composite fiber layer has a pore size of 1-100$\mu$m.

The present disclosure further relates to the use of the tissue repair scaffold, said tissue repair scaffold is used as brain dura matter repairing material, spinal dura matter repairing material, tissue engineering scaffold material, artificial skin, wound dressing, a biological membrane, a wound cladding material, a hemostatic material, a postoperative adhesion prevention material or a plastic and aesthetic material.

The present disclosure further relates to the preparation method for the tissue repair scaffold, wherein the composite fiber layer of the tissue repair scaffold is prepared through following steps: dissolving the adhesion factor and the hydrophobic synthetic material in the same kind of solvent; and then obtaining a membrane containing the composite fibers through the preparation technique of electrostatic spinning, centrifugal spinning, melt blowing, melt electrospinning, or a combination thereof, said solvent used in the steps of preparing the composite fiber layer preferably at least comprising one kind of fluorine-containing solvent, said fluorine-containing solvent preferably including hexafluoroisopropanol, trifluoroethanol, or trifluoroacetic acid.

The preparation method, further comprising a step of preparing the porous 3D reticulate structure by immersing the prepared membrane containing composite fiber into a solution of hydrophilic material, taking out the membrane, and freeze-drying the membrane.

In the preparation method, the solvent used in the step of preparing composite fiber layer at least comprises one kind of fluorine-containing solvent.

In the preparation method, the fluorine-containing solvents include hexafluoroisopropanol, trifluoroethanol, trifluoroacetic acid.

Advantageous effects

The tissue repair scaffold of the present disclosure has unique structure, higher porosity and specific surface area; the growth of the cells into the scaffold is promoted, and the repair rate of the newly generated tissue is accelerated.

After implantation, the scaffold form proper attachment to the tissue anatomical structure through physical force; the body fluid in the tissue around is rapidly absorbed, a connection with tissue is further formed through the biologically induced adhesive effect; along with the scrawling and ingrowth of the collagen fibers, the scaffold material integrates with the tissue around to achieve repair.

The composite fibers formed with different material differ in the controlled degradation rate. The space left through the degradation of the material which degrades faster is advantageous for the fibroblasts to grow in, while the material which degrades slowly may prevent the defect formation due to the fast degradation of the material at the early stage, to ensure the sufficient mechanical support before complete repair.

The tissue repair scaffold of the present disclosure prepared with biomimetic technology has large specific surface area as a feature of nanofibers; the tissue repair scaffold is advantageous for the adhesion and proliferation of the cells, and has favorable compliance and attachment to the tissue. The requirements for a tissue repair scaffold, in consideration of biological and mechanical properties, are fulfilled. In addition, the surface topological structure of the scaffold is advantageous for directing the differentiation of the cells. Due to the light and soft texture of the tissue repair scaffold of the present disclosure, the comfortable feeling of the patients is improved, the trauma of the patients is relieved, and the recovery of the patient is facilitated.

In an embodiment of the present disclosure, the composite fibers formed with different materials are firstly prepared through biomimetic technology. The composite fibers contain both hydrophobic material and hydrophilic material. In the solution of hydrophilic material, the composite fiber layer swells, and the dimensional structure thereof expands, therefore the hydrophilic material may penetrate into the composite fiber layer. Then the swollen structure is fixed through techniques such as freeze-drying, and the novel unique porous dimensional structure and surface structure are achieved, the porous 3D reticulate structure where multiple structures simultaneously exist is formed. The hydrophobic material degrades slowly; therefore it maintains favorable mechanical support function at the early stage of implantation. The combination of hydrophobic material and hydrophilic material generates favorable compatibility and attachment to the tissue around, therefore advantageous repair effect is achieved.

Other features and aspects of the present disclosure will become clear through the detailed description to the exemplary examples with reference to the accompanying drawings.

**Brief Description of the Drawings**

The drawings which are incorporated in and constitute a part of this specification, together with the specification, shows the exemplary examples, features and aspects of the present disclosure, and are used for illustrating the principle of the present disclosure.

Figure 1A and 1B are SEM image of the composite fibers formed with adhesion factors and hydrophobic synthetic materials used in the composite fiber layer, and SEM image of the composite fibers after being treated with a solvent which is capable of dissolving one of the materials, respectively.

Figure 2 is an SEM image of the surface of the composite fiber layer of the present disclosure.

Figure 3 is an SEM image showing the porous 3D reticulate structure at the surface of the tissue repair scaffold of the present disclosure (10 $\mu$m).

Figure 4 is an SEM image showing the porous 3D reticulate structure at the surface of the tissue repair scaffold of the present disclosure (1$\mu$m).

Figure 5 is an SEM image of the cross section of the tissue repair scaffold of the present disclosure (20 $\mu$m).

Figure 6 is an image showing the attachment of the tissue repair scaffold to the brain tissue in Experimental Example 1 of the present disclosure.

Figure 7 is an image showing the attachment of the tissue repair scaffold to the brain tissue in Experimental Comparative Example 1 of the present disclosure.

Figure 8 is an image of a pathological section in Experimental Example 1 of the present disclosure.

Figure 9 is an image of a pathological section in Experimental Example 2 of the present disclosure.

**Description of the Embodiments**

More details are provided in the following embodiments to better illustrate the present disclosure. However, it should be understood that those skilled in the arts are able to implement the present disclosure even without certain details in the specification.

In one embodiment of the present disclosure, the tissue repair scaffold comprises a surface of at least one layer of composite fiber layer(s), said composite fiber layer comprises composite fibers formed with an adhesion factor and a hydrophobic synthetic material.

In another embodiment, the tissue repair scaffold has a porous 3D reticulate structure formed by a composite fiber layer and a hydrophilic material; said composite fiber layer comprises composite fibers formed with an adhesion factor and a hydrophobic synthetic material.

**First embodiment**

In this embodiment, the tissue repair scaffold comprises a surface of at least one layer of composite fiber layer(s), said composite fiber layer comprises composite fibers formed with an adhesion factor and a hydrophobic synthetic material. The tissue repair scaffold may be formed with only one layer of composite fiber layer, in this instance the tissue repair scaffold is substantially a "composite fiber membrane". The "composite fiber membrane", when used as a layer of the tissue repair scaffold, is referred to as a "composite fiber layer". Therefore it should be understood that the "composite fiber membrane" and the "composite fiber layer" in the present disclosure are synonyms only differ in nomenclature; they possess the same structure and composition, both comprise composite fibers formed with adhesion factors and hydrophobic synthetic materials.

<Composite fiber layer>

The composite fiber layer of the present disclosure comprises composite fibers formed with an adhesion factor and a hydrophobic material.

The adhesion factor of the present disclosure refers to a material that, when attaches to the tissue, enables the tissue repair scaffold to acquire favorable attachment to the tissue and form tissue adhesive force, comply with the anatomical structure, and subsequently absorb the coagulation substances to form bioadhesion; particularly, the adhesion factor of the present disclosure is a hydrophilic material which contains one or more group(s) selected from an amino group, an imino group, a nitrilo group, an amide group, a polypeptide, an ester group, a hydroxy group, a carboxy group and an ether group.

The tissue adhesive force according to the present disclosure includes physical force and/or biological adhesive force generated by the adhesion factors. The physical force included in the tissue adhesive force refers to the friction force between the scaffold and the tissue, which is generated when the material adsorbs the hollow and gaps at the surface of the tissue to achieve attachment, the effective contact area is increased (which would be larger than 60%, compared with that of the membrane formed with hydrophobic materials), and favorable attachment to the tissue is formed. The physical force included in the tissue adhesive force according to the present disclosure is in the range of 0.5-10 N, preferably 0.8-8 N, more preferably larger than 1 N. The method for the measurement of the physical force is as follows: the scaffold material in membrane form is cut into a strip with a size of 10mm*60mm, infiltrated with water, and the water at the surface is absorbed with a napkin; a rabbit skin strip with a size of 10mm*60mm is taken; the two strips (scaffold material and rabbit skin) are symmetrically, intersectly overlapped at their both ends to form a strip with a size of 10mm*70mm; then the maximal force recorded when the relative movement between the overlapped scaffold material strip and rabbit skin strip occurs is measured with a tension testing machine.

The biological adhesive force is a kind of relatively strong biological sticking force generated in such a case that, the adhesion factor of the present disclosure may rapidly absorb various coagulation proteins in the around tissue fluid; once reach the inner part of the scaffold, the coagulation proteins promote the conversion of prothrombin into thrombin, and the conversion of fibrinogen into fibrin, thus the coagulation process is accelerated, results in the adhesion with the tissue around. The biological adhesive force can be characterized through the adhesion experiment: a white rabbit is anaesthetized with pentobarbital sodium, denuded; on the skin, a 5cm*7cm wound showing uniform dotted bleeding is created; a piece of material with a size of 4cm*6cm is attached to the bleeding wound, compressed with a pressure of 40 mmHg for 5 min; a tension transducer (Type JN-IB-100) and a double channel physiological recorder (LMS-2B) are connected; the material is dragged upward vertically with a constant velocity; the sticking force is calculated based on the peak value of the maximal traction force. The sticking force is larger than 1.001 g/cm$^2$.

In addition, the adhesion factors of the present disclosure may rapidly absorb various proteins and nutrients from the tissue fluid around into the inner part of the scaffold, to increase the rate that the collagen fibers grow into the scaffold, and promote the ingrowth of the newly generated tissue. The adhesion factors of the present disclosure have multiple functions such as adhesion, coagulation, promotion of healing, inhibition of hyperplastic scar, etc.

The specific examples of the adhesion factors of the present disclosure include but are not limited to: proteins with certain degree of hydrophilicity, and derivative materials thereof, e.g. collagen, fibrin, silk proteins, elastin-like peptide polymers, gelatin; cellulose with certain degree of hydrophilicity, and derivative materials thereof, e.g. cellulose, modified cellulose, starch, cellulose; hydrophilic alcohols, ethers and derivative materials thereof, e.g. polyvinyl alcohol, polyethylene glycol (PEG), block polyethers like polyether F127, polyether P123; chitosan and derivative materials thereof, e.g. chitosan, modified chitosan; saccharides and derivative materials, e.g. heparin, dextran, alginic acid, agar, chondroitin sulfate; nitrogen-containing hydrophilic substances, e.g. polymers like hydrophilic polyurethane, polyvinylpyrrolidone. Among these, chitosan, modified chitosan, fibrin, silk proteins, elastin-like peptide polymers, polyvinyl alcohol, polyvinylpyrrolidone, collagen and derivatives thereof, gelatin are preferably used.

The hydrophobic materials of the present disclosure mainly include hydrophobic polyurethane, polyethylene terephthalate, polycaprolactone (PCL), polyglycolic acid (PGA), poly(lactic acid-glycolic acid) copolymer (PLGA), polymer of 1,3-propylene glycol (PDO), poly(lactic acid-caprolactone) copolymers (PLC), polylactic acid (PLA), etc. Among these, PLA, PCL, PGA, PLGA, PLC are preferably used.

The adhesion factors and hydrophobic material of the present disclosure may be combined in any form to compose composite fibers. The adhesion factors may be existed inside and/or at the surface of the composite fibers. The composite fibers may have one or more kinds of structure selected from core structure, crossover arranged structure, block structure. These structures can be adjusted by means of regulating the ratio between adhesion factor and hydrophobic synthetic material, or the preparation condition, and so on.

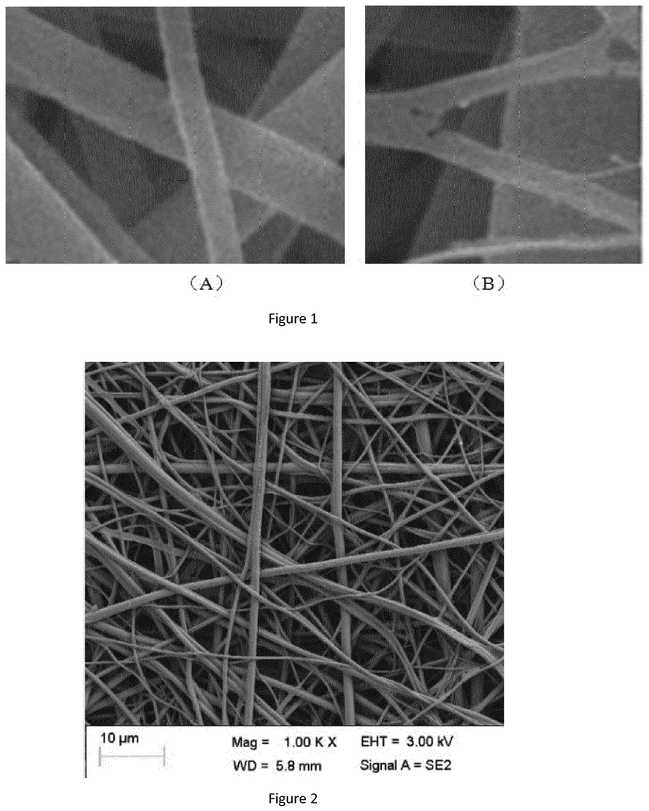

The structure of the composite fibers of the present disclosure can be characterized by SEM images. As shown in Figure 1, through the comparison between an SEM image of composite fibers and an SEM image showing the composite fibers after being treated with a solvent which can dissolve one of the material in the fiber, the composite structure of the material can be clearly observed. Figure 1-A is an SEM image of the composite fibers of the present disclosure. When the composite fibers are treated with a solvent, said solvent can dissolve only one material in the composite but can not dissolve the other material, the fibrous filament residue is obtained, where a part of the composite filament has been dissolved, while the other part are not dissolved, as shown in Figure 1-B. Figure 2 is an SEM image of the surface of composite fiber layer of the present disclosure, from which a crossover structure of the fibrous filaments can be clearly observed.

The hydrophilic materials and hydrophobic materials for tissue repair scaffold provided in the present disclosure have different intrinsic degradation rate. Through regulating the ratio between adhesion factors and hydrophobic synthetic materials in the surface, the rate that the tissue fluid penetrate into the scaffold can be controlled, then the degradation rate of the surface and the inner part of the scaffold is under control. It may help prevent tissue repair failure, which occurs in such a situation that the material degrades too fast while the newly generated tissue has not completely cover the defect yet. The rate of material degradation and the rate that newly generated tissue grows into the scaffold can also be balanced. The degradation of the material which degrades faster leaves space for the newly generated tissue, which is favorable for the crawling and replacement of the newly generated tissue, meanwhile the material which degrades slower may provide certain degree of mechanical support at the initial stage of tissue repair.

In the composite fiber of the present disclosure, the content of the adhesion factor is 5%-95% by mass, preferably 10%-50%, more preferably 15%-30%.

The surface of the composite fiber layer of the present disclosure has small contact angle ($\theta \leq 90°$ or $\theta = 0$, where the liquid is water).

The water absorption ratio of the composite fiber membrane of the present disclosure is larger than 100%, where the water absorption ratio is calculated through the following method:

Water absorption ratio = [(the mass of the membrane after water absorption and removal of water at the surface with napkin – the mass of the membrane before water absorption)/the mass of the membrane]*100%.

Furthermore, the composite fiber membrane of the present disclosure has a hydrophilic-lipophilic balance (HLB) value of 1-3, preferably 1-6. HLB value is a measure that describes the level of balance in size and strength between the hydrophilic groups and lipophilic groups in the surfactant molecule, defined as the hydrophilic-lipophilic balance of the surfactant. The lipophilic or hydrophilic degree of a surfactant can be determined with the value of HLB. A larger HLB value indicates stronger hydrophilicity, while a smaller HLB value indicates a stronger lipophilicity. In general, the HLB value varies in the range of 1-40. In practice, HLB serves as important reference. A lipophilic surfactant has lower HLB, while a hydrophilic surfactant has higher HLB. The HLB for hydrophilic-lipophilic transition is 10. When the HLB value is smaller than 10 means lipophilic, and when the HLB value is larger than 10 means hydrophilic.

The hydrophobic synthetic material contained in the tissue repair scaffold as provided in the present disclosure may prevent the adsorption of the proteins to a certain extent, therefore play a role in preventing the adhesion to the tissue. Once water is absorbed, the adhesion factor swells and makes the scaffold softer. The attachment to the tissue is promoted; the scaffold forms compliant attachment to the tissue anatomical structure, and the attachment to the tissue has favorable strength.

The composite fiber layer of the present disclosure can be prepared through the following method: the adhesion factor and the hydrophobic material are dissolved in the same kind of solvent; then a layer containing the composite fiber is obtained by means of electrostatic spinning, centrifugal spinning, melt blowing, melt electrospinning, or a combination thereof, wherein the electrostatic spinning technology, the centrifugal spinning technology, the melt blowing technology, or the melt electrospinning technology are preferably used, and the centrifugal spinning technology or the electrostatic spinning technology are more preferably used.

The composite fiber layer of the present disclosure can be prepared with the centrifugal spinning technology. The hydrophobic synthetic material and the adhesion factor can be simultaneously dissolved in the same kind of solvent to form a homogenous solution. The solution enters into a centrifugal spinning machine via a propel pump. The propelling rate of the propel pump, the velocity of centrifugal coiling, and the temperature for spinning are adjusted, so that the solution is spun into fibers rapidly. Once the procedure is completed, the membrane is taken from the receiver, and the solvent is removed by vacuum drying.

The composite fiber layer of the present disclosure can also be prepared through electrostatic spinning technology. The hydrophobic material and adhesion factor are simultaneously dissolved in the same kind of solvent to form a homogenous solution. The solution enters into an electrostatic spinning machine via a propel pump. The parameters like the propelling rate of the propel pump, the velocity of the rotating roller, the spinning voltage, the receiving distance, etc., are adjusted so that the solution is spun into fibers rapidly. Once the procedure is completed, the membrane is taken from the receiver, and the solvent is removed by vacuum drying.

In the preparation of the composite fiber layer of the present disclosure, the solvent used contains at least one kind of fluorine-containing solvent, said fluorine-containing solvent includes hexafluoroisopropanol, trifluoroethanol, trifluoroacetic acid and the like.

Preferably, the composite fiber layer is formed through interweaving composite fiber filaments with diameter of 10nm~100μm, and the composite fiber layer has a porous structure.

**Second embodiment**

In this embodiment, the tissue repair scaffold has a porous 3D reticulate structure formed by a composite fiber layer and a hydrophilic material; said composite fiber layer contains composite fibers formed with adhesion factors and hydrophobic synthetic materials. Wherein the composite fiber layer is the same with that described in the first embodiment above.

<Porous 3D reticulate structure>

In this embodiment, the tissue repair scaffold has a porous 3D reticulate structure formed by a composite fiber layer and a hydrophilic material. The porous 3D structure is formed on and/or inside the surface of the composite fiber layer.

The porous 3D reticulate structure can be formed through the steps of: immersing the prepared composite fiber layer (membrane) into a solution of hydrophilic material; taking out the composite fiber layer; and freeze-drying the composite fiber layer.

The hydrophilic material used for forming the porous 3D reticulate structure may be proteins with certain degree of hydrophilicity and the derivative materials thereof, e.g. collagen, fibrin, silk proteins, elastin-like peptide polymers, gelatin; cellulose with certain degree of hydrophilicity, and derivative materials thereof, e.g. cellulose, modified cellulose, starch, cellulose; hydrophilic alcohols, ethers and derivative materials thereof, e.g. polyvinyl alcohol, polyethylene glycol (PEG), block polyethers like polyether F127, polyether P123; chitosan and derivative materials thereof, e.g. chitosan, modified chitosan; saccharides and derivative materials thereof, e.g. heparin, dextran, alginic acid, agar, chondroitin sulfate; nitrogen-containing hydrophilic substances, e.g. polymer materials like hydrophilic polyurethane, polyvinylpyrrolidone. Among these, chitosan, modified chitosan, fibrin, silk proteins, elastin-like peptide polymers, polyvinyl alcohol, polyvinylpyrrolidone, collagen and derivatives thereof, gelatin are preferably used. The concentration of the solute in the solution is not particularly limited. When the concentration is low, a porous 3D structure with relatively large pores can be formed on and/or inside the surface of the composite fiber layer; when the concentration is high, a porous 3D reticulate structure with relatively small pores can be formed on and/or inside the surface of the composite fiber layer. The concentration of the solution is preferably 0.1%-30% by mass percentage, and more preferably 0.5-10% by mass percentage.

The solvent in the solution of the hydrophilic material is not particularly limited; it can be a routine solvent that can dissolve the hydrophilic materials indicated above.

The immersion according to the present disclosure includes routine immersion method, ultrasonic immersion and other auxiliary immersion method. The freeze-drying method according to the present disclosure includes the methods wherein the equipment such as vacuum freeze dryer, freeze dryer, vacuum scraper dryer, freeze air dryer and the like is used in manufacture.

<Tissue repair scaffold>

In the embodiment, the tissue repair scaffold can be prepared through the following procedure. Firstly a composite fiber layer (membrane) is prepared. Then the composite fiber layer (membrane) is immersed in a solution of water-soluble materials, taken out, and treated in the freeze-drying process.

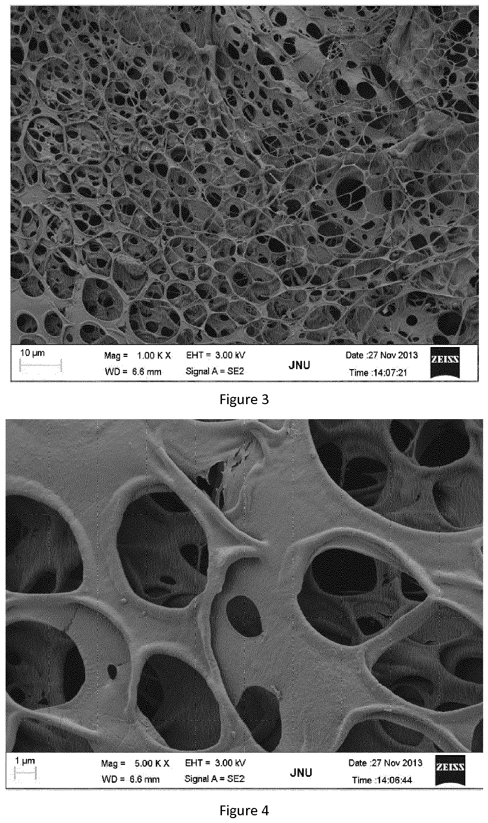

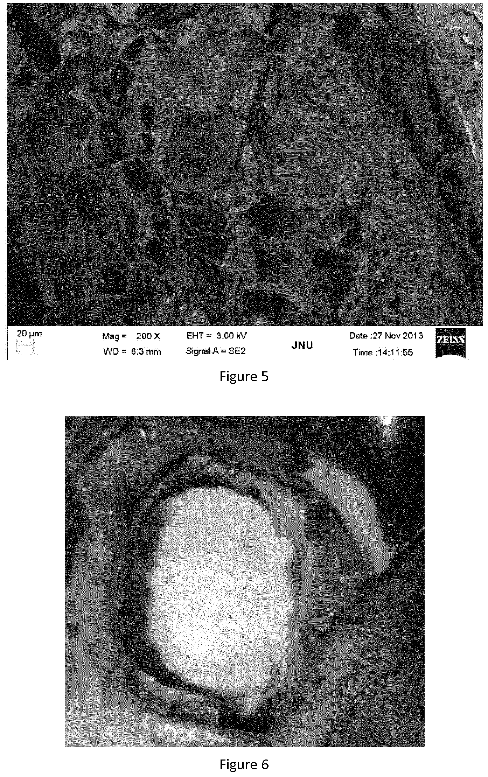

In this embodiment, the porous 3D reticulate structure of the tissue repair scaffold, which is formed with a composite fiber layer and a hydrophilic material, or in other words the honeycomb-shape porous structure, is shown in the SEM images of Figure 3 and 4. The cross section of the tissue repair scaffold is shown in Figure 5. The diameter of the pores is determined according to the method described in GTT TM 017-2012.

In this embodiment, once the tissue repair scaffold adheres to the tissue around, the burst strength is 0.10 kPa or more, more preferably 0.10-30 kPa. The burst strength is measured through the following procedure. The rabbit skin is taken and cut into a circular patch with a diameter of 3 cm, and at the center of the patch, a hole with a diameter of 1 cm is made. The tissue repair scaffold of the present disclosure is taken and cut into a circular patch with a diameter of 3 cm, immersed, then attached to the surface of the rabbit skin in such a way that the tissue repair scaffold evenly covers the surface of the hole. Then the rabbit skin, which is covered with the scaffold, is used to seal the opening of the container in which a pressure sensor is installed, to ensure the tight seal of the container. The air is evenly blown into the container, and when the tissue repair scaffold is forced open, the reading of the pressure sensor is recorded as the burst strength.

The tissue repair scaffold has extremely fast water absorption rate. If the tissue repair scaffold is immersed in water for 10 s, the water absorption ratio thereof may reach 100%-5000%, preferably 100%-1000%, more preferably 100%-500%.

The method for calculating the water absorption ratio of the tissue repair scaffold is the same as that for calculating the water absorption ratio of the composite fiber membrane. The physical force and biological adhesive force of the tissue repair scaffold of the present disclosure can be measured in the same way as that used for the composite fiber membrane.

The tissue repair scaffold has very small contact angle ($\theta < 90°$ or $\theta = 0$, where the liquid is water).

In the tissue repair scaffold, the total content of the hydrophilic materials is 5%-95% by mass.

The tissue repair scaffold of the present disclosure can be used as dura matter repairing material, a tissue engineering scaffold material, artificial skin, a wound dressing, a biological membrane, a wound cladding material, a hemostatic material, a postoperative adhesion prevention material, and a plastic and aesthetic material.

## Examples

<Preparation of the tissue repair scaffold in the first embodiment

### Example 1

PCL and gelatin were dissolved in trifluoroethanol in a mass ratio of 10:1 to form a solution. The solution entered into a centrifugal spinning machine via a propel pump. The propelling rate of the propel pump was 150 g/min; the velocity of centrifugal coiling was 6000 m/min. The solution was rapidly spun into fibers at a spinning temperature of 50°C. Once the procedure was completed, the membrane was taken from the receiver, and the solvent was removed by vacuum drying, so that a composite fiber membrane was obtained.

An SEM image of the composite fibers is shown in Figure 1-A. After being treated with water at a temperature of 50°C, which may dissolve gelatin but may not dissolve PCL, the composite fiber has an SEM image as shown in Figure 1-B. It can be clearly observed that the composite fibers have a composite structure formed by PCL and gelatin.

### Example 2

PLLA and chitosan were dissolved in trifluoroacetic acid in a mass ratio of 10:9 to form a solution. The solution entered into a centrifugal spinning machine via a propel pump. The propelling rate of the propel pump was 100 g/min; the velocity of centrifugal coiling was 3000 m/min. The solution was rapidly spun into fibers at a spinning temperature of 80°C. Once the procedure was completed, the membrane was taken from the receiver, and the solvent was removed by vacuum drying, so that a composite fiber membrane was obtained.

### Example 3

PCL and cellulose acetate were dissolved in hexafluoroisopropanol in a mass ratio of 2:1 to form a solution, then the solution was put into an electrostatic spinning syringe. The injection rate of the micro-injection pump was adjusted to 3 mL/hour. The voltage of the high-pressure generator was adjusted to 20 kV. The receiving distance of the receiving device was adjusted to 19 cm. A rotating roller was used as the receiving device; the movement speed of the electrospinning needle was 10 cm/s, while the rotation speed of the receiving roller was 100rpm. The electrospinning was performed with two needles simultaneously for 8 hours, and a membrane with a single layer structure was obtained. The membrane was taken from the receiving roller, and the solvent was removed by vacuum drying, so that a composite fiber membrane was obtained.

Example 4

PLLA and collagen were dissolved in hexafluoroisopropanol in a mass ratio of 4:1 to form a solution, then the solution was put into an electrostatic spinning syringe. The injection rate of the micro-injection pump was adjusted to 4 mL/hour. The voltage of the high-pressure generator was adjusted to 23 kV. The receiving distance of the receiving device was adjusted to 19 cm. A rotating roller was used as the receiving device; the movement speed of the electrospinning needle was 15 cm/s, while the rotation speed of the receiving roller was 120rpm. The electrospinning was performed with two needles simultaneously for 8 hours, and a membrane with a single layer structure was obtained. The membrane was taken from the receiving roller, and the solvent was removed by vacuum drying, so that a composite fiber membrane was obtained.

Example 5

PLGA and chondroitin sulfate were dissolved in trifluoroacetic acid in a mass ratio of 4:1 to form a solution, then the solution was put into an electrostatic spinning syringe. The injection rate of the micro-injection pump was adjusted to 5 mL/hour. The voltage of the high-pressure generator was adjusted to 24 kV. The receiving distance of the receiving device was adjusted to 19 cm. A rotating roller was used as the receiving device; the movement speed of the electrospinning needle was 10 cm/s, while the rotation speed of the receiving roller was 150rpm. The electrospinning was performed with two needles simultaneously for 8 hours, and a membrane with a single layer structure was obtained. The membrane was taken from the receiving roller, and the solvent was removed by vacuum drying, so that a composite fiber membrane was obtained.

Example 6

PLLA and gelatin were dissolved in hexafluoroisopropanol in a mass ratio of 3.5:1 to form a solution, then the solution was put into an electrostatic spinning syringe. The injection rate of the micro-injection pump was adjusted to 7 mL/hour. The voltage difference of the high-pressure generator was adjusted to 28 kV. The receiving distance of the receiving device was adjusted to 21 cm. A rotating roller was used as the receiving device; the movement speed of the electrospinning needle was 10 cm/s, while the rotation speed of the receiving roller was 80rpm. The electrospinning was performed with two needles simultaneously for 8 hours, and a membrane with a single layer structure was obtained. The membrane was taken from the receiving roller, and the solvent was removed by vacuum drying, so that a composite fiber membrane was obtained.

Example 7

PLLA and polyether F127 were dissolved in hexafluoroisopropanol in a mass ratio of 3:1 to form a solution, then the solution was put into an electrostatic spinning syringe. The injection rate of the micro-injection pump was adjusted to 9 mL/hour. The voltage difference of the high-pressure generator was adjusted to 30 kV. The receiving distance of the receiving device was adjusted to 23 cm. A rotating roller was used as the receiving device; the movement speed of the electrospinning needle was 10 cm/s, while the rotation speed of the receiving roller was 200rpm. The electrospinning was performed with two needles simultaneously for 8 hours, and a membrane with a single layer structure was obtained. The membrane was taken from the receiving roller, and the solvent was removed by vacuum drying, so that a composite fiber membrane was obtained.

Example 8

PLC and polyether P123 were dissolved in hexafluoroisopropanol in a mass ratio of 1:1 to form a solution, then the solution was put into an electrostatic spinning syringe. The injection rate of the micro-injection pump was adjusted to 10 mL/hour. The voltage difference of the high-pressure generator was adjusted to 33 kV. The receiving distance of the receiving device was adjusted to 25 cm. A rotating roller was used as the receiving device; the movement speed of the electrospinning needle was 10 cm/s, while the rotation speed of the receiving roller was 100rpm. The electrospinning was performed with two needles simultaneously for 8 hours, and a membrane with a single layer structure was obtained. The membrane was taken from the receiving roller, and the solvent was removed by vacuum drying, so that a composite fiber membrane was obtained.

Example 9

PLC and PEG were dissolved in hexafluoroisopropanol in a mass ratio of 1:2 to form a solution, then the solution was put into an electrostatic spinning syringe. The injection rate of the micro-injection pump was adjusted to 6 mL/hour. The voltage difference of the high-pressure generator was adjusted to 27 kV. The receiving distance of the receiving device was adjusted to 17 cm. A rotating roller was used as the receiving device; the movement speed of the electrospinning needle was 10 cm/s, while the rotation speed of the receiving roller was 160rpm. The electrospinning was performed with two needles simultaneously for 8 hours, and a membrane with a single layer structure was obtained. The membrane was taken from the receiving roller, and the solvent was removed by vacuum drying, so that a composite fiber membrane was obtained.

Example 10

PLC and hydrophilic polyurethane were dissolved in hexafluoroisopropanol in a mass ratio of 1:3 to form a solution, then the solution was put into an electrostatic spinning syringe. The injection rate of the micro-injection pump was adjusted to 6 mL/hour. The voltage difference of the high-pressure generator was adjusted to 26 kV. The receiving distance of the receiving device was adjusted to 15 cm. A rotating roller was used as the receiving device; the movement speed of the electrospinning needle was 10 cm/s, while the rotation speed of the receiving roller was 300 rpm. The electrospinning was performed with two needles simultaneously for 8 hours, and a membrane with a single layer structure was obtained. The membrane was taken from the receiving roller, and the solvent was removed by vacuum drying, so that a composite fiber membrane was obtained.

<Preparation of the tissue repair scaffold in the second embodiment

Example 11

PCL and collagen were dissolved in hexafluoroisopropanol in a mass ratio of 4:1 to form a solution with total concentration of 8% by mass. Then the solution entered into a centrifugal spinning machine via a propel pump. The propelling rate of the propel pump was 150 g/min; the velocity of centrifugal coiling was 6000 m/min. The solution was rapidly spun into fibers at a spinning temperature of 50°C. Once the procedure was completed, a membrane was taken from the receiver, and the remaining solvent was removed to obtain a composite fiber membrane (designated as scaffold A). The composite fiber membrane thus obtained was immersed into a gelatin aqueous solution (2% by mass) for 5 min, taken out, and freeze-drying was performed, so that a tissue repair scaffold was obtained (designated as scaffold B).

Example 12

PLLA and gelatin were dissolved in hexafluoroisopropanol in a mass ratio of 2:1 to form a solution with total concentration of 8% by mass, then the solution was put into an electrostatic spinning syringe. The injection rate of the micro-injection pump was adjusted to 4mL/hour. The voltage of the high-pressure generator was adjusted to 23 kV. The receiving distance of the receiving device was adjusted to 19 cm. A rotating roller was used as the receiving device; the movement speed of the electrospinning needle was 15 cm/s, while the rotation speed of the receiving roller was 120 rpm. The electrospinning was performed with two needles simultaneously for 8 hours. Once the procedure is completed, the membrane is taken from the receiving roller, and the remaining solvent in the membrane was removed. The membrane was immersed into a gelatin aqueous solution (7% by mass) for 30 min, taken out, and freeze-drying was performed, so that a tissue repair scaffold was obtained (designated as scaffold C).

Example 13

PLGA and chondroitin sulfate were dissolved in trifluoroacetic acid in a mass ratio of 4:1 to form a solution with total concentration of 8% by mass, then the solution was put into an electrostatic spinning syringe. The injection rate of the micro-injection pump was adjusted to 5 mL/hour. The voltage of the high-pressure generator was adjusted to 24 kV. The receiving distance of the receiving device was adjusted to 19 cm. A rotating roller was used as the receiving device; the movement speed of the electrospinning needle was 10 cm/s, while the rotation speed of the receiving roller was 150 rpm. The electrospinning was performed with two needles simultaneously for 8 hours. Once the procedure is completed, the membrane is taken from the receiving roller, and the remaining solvent in the membrane was removed. The membrane was immersed into a gelatin aqueous solution (2% by mass) for 10 min, taken out, and freeze-drying was performed, so that a tissue repair scaffold was obtained.

Example 14

PLLA and polyether F127 were dissolved in hexafluoroisopropanol in a mass ratio of 3:1 to form a solution with total concentration of 8% by mass, then the solution was put into an electrostatic spinning syringe. The injection rate of the micro-injection pump was adjusted to 9 mL/hour. The voltage difference of the high-pressure generator was adjusted to 30 kV. The receiving distance of the receiving device was adjusted to 23 cm. A rotating roller was used as the receiving device; the movement speed of the electrospinning needle was 10 cm/s, while the rotation speed of the receiving roller was 200 rpm. The electrospinning was performed with two needles simultaneously for 8 hours. Once the procedure is completed, a membrane is taken from the receiving roller, and the remaining solvent in the membrane was removed. The membrane was immersed into a gelatin aqueous solution (3% by mass) for 5 min, taken out, and freeze-drying was performed, so that a tissue repair scaffold was obtained.

Example 15

PLC and PEG were dissolved in hexafluoroisopropanol in a mass ratio of 1:2 to form a solution with total concentration of 8% by mass, then the solution was put into an electrostatic spinning syringe. The injection rate of the micro-injection pump was adjusted to 6mL/hour. The voltage difference of the high-pressure generator was adjusted to 27 kV. The receiving distance of the receiving device was adjusted to 17 cm. A rotating roller was used as the receiving device; the movement speed of the electrospinning needle was 10 cm/s, while the rotation speed of the receiving roller was 160 rpm. The electrospinning was performed with two needles simultaneously for 8 hours. Once the procedure is completed, a membrane is taken from the receiving roller, and the remaining solvent in the membrane was removed. The membrane was immersed into a gelatin aqueous solution (5% by mass) for 3 min, taken out, and freeze-drying was performed, so that a tissue repair scaffold was obtained.

Example 16

PLC and hydrophilic polyurethane were dissolved in hexafluoroisopropanol in a mass ratio of 1:3 to form a solution with total concentration of 8% by mass, then the solution was put into an electrostatic spinning syringe. The injection rate of the micro-injection pump was adjusted to 6 mL/hour. The voltage difference of the high-pressure generator was adjusted to 26 kV. The receiving distance of the receiving device was adjusted to 15 cm. A rotating roller was used as the receiving device; the movement speed of the electrospinning needle was 10 cm/s, while the rotation speed of the receiving roller was 300 rpm. The electrospinning was performed with two needles simultaneously for 8 hours. Once the procedure is completed, a membrane is taken from the receiving roller, and the remaining solvent in the membrane was removed. The membrane was immersed into a gelatin aqueous solution (6% by mass) for 3min, taken out, and freeze-drying was performed, so that a tissue repair scaffold was obtained.

<Preparation in Comparative Examples>

Comparative Example 1

A membrane was prepared through the same process as that used in Example 1, except that a mixed material of PCL and gelatin was replaced by only PCL. The sample prepared was numbered as D (designated as membrane D). A membrane was prepared through the same process as that used in Example 3, except that a mixed material of PCL and cellulose acetate was replaced by only PLLA. The sample prepared was numbered as E (designated as membrane E). A membrane was prepared through the same process as that used in Example 4, except that a mixed material of PLLA and collagen was replaced by only PLLA. The sample prepared was numbered as F (designated as membrane F).

The composite fiber membranes obtained in Example 1-10, and the fiber membranes D, E, F were tested for biological adhesive force and physical force. The method and result of the test are shown below.

The method for the measurement of physical force: the composite fiber membranes obtained in Example 1-10, and the fiber membranes D, E, F were cut into strips with a size of 10mm*60mm respectively, infiltrated with water, and then the water at the surface was absorbed with a napkin; the rabbit skin in strip shape with a size of 10mm*60mm was taken; the cut membrane and the rabbit skin were intersectly overlapped at their both ends to form a strip with a size of 10mm*70mm. Then the overlapped strip sheet material was tested with a tension testing machine (HY3080). When the relative movement between the strip sheet and the rabbit skin occurred, the maximal tensile force was measured.

The method for the measurement of biological adhesive force: a white rabbit was anaesthetized with pentobarbital sodium, denuded; on the skin, a 5 cm*7 cm wound showing uniform dotted bleeding was created. The composite fiber membranes obtained in Example 1-10, and the fiber membranes D, E, F were cut into materials with a size of 4cm*6cm respectively, and attached to the bleeding wound, compressed with a pressure of 40 mmHg for 5 min. The material was dragged upward vertically with a constant velocity. A tension transducer (Type JN-IB-100) and a double channel physiological recorder (LMS-2B) were connected, and the biological adhesive force was calculated based on the peak value of the maximal traction force.

Table 1

| Serial No. of the sample | Physical force (N) | Biological adhesive force (g/cm$^2$) |
|---|---|---|
| Membrane D | NA* | NA |
| Membrane E | NA | NA |
| Membrane F | NA | NA |
| Composite fiber membrane in Example 1 | 1.021 | 1.11 |
| Composite fiber membrane in Example 2 | 1.061 | 1.72 |
| Composite fiber membrane in Example 3 | 1.055 | 1.64 |
| Composite fiber membrane in Example 4 | 1.041 | 1.21 |
| Composite fiber membrane in Example 5 | 1.042 | 1.32 |
| Composite fiber membrane in Example 6 | 1.049 | 1.47 |
| Composite fiber membrane in Example 7 | 1.052 | 1.55 |
| Composite fiber membrane in Example 8 | 1.065 | 1.84 |
| Composite fiber membrane in Example 9 | 1.069 | 1.92 |
| Composite fiber membrane in Example 10 | 1.073 | 2.01 |
| *NA indicates that the force is too small to be detected. | | |

Comparative Example 2

PCL was dissolved in hexafluoroisopropanol at a concentration of 8% to form a solution. Then the solution entered into a centrifugal spinning machine via a propel pump. The propelling rate of the propel pump was 150 g/min; the velocity of centrifugal coiling was 6000 m/min. The solution was rapidly spun into fibers at a spinning temperature of 50°C. Once the procedure was completed, a membrane was taken from the receiver, and the remaining solvent was removed from the material. The material was immersed into a gelatin aqueous solution (2% by mass) for 5 min. The inner part of the membrane material could not be infiltrated by water. The material was taken out, and directly freeze-dried, so that a tissue repair scaffold was obtained (designated as scaffold G).

Comparative Example 3

Collagen was dissolved in hexafluoroisopropanol at a concentration of 8% to form a solution. Then the solution entered into a centrifugal spinning machine via a propel pump. The propelling rate of the propel pump was 150 g/min; the velocity of centrifugal coiling was 6000 m/min. The solution was rapidly spun into fibers at a spinning temperature of 50°C. Once the procedure was completed, a membrane was taken from the receiver, and the remaining solvent was removed from the material. The material was immersed into a gelatin aqueous solution (3% by mass) for 15 min. The material was taken out, and directly freeze-dried, so that a tissue repair scaffold was obtained (designated as scaffold H).

Table 2 Comparison of performance of scaffolds with different materials

| Scaffold | Tensile strength (MPa) | Water absorption ratio in 10 s | Physical force (N) | Biological adhesive force (g/cm$^2$) | Burst strength (kPa) |
|---|---|---|---|---|---|
| A | 2.6 | 110% | 1.03 | 1.21 | 0.15 |

(continued)

| Scaffold | Tensile strength (MPa) | Water absorption ratio in 10 s | Physical force (N) | Biological adhesive force (g/cm$^2$) | Burst strength (kPa) |
|---|---|---|---|---|---|
| B | 2.4 | 200% | 1.12 | 1.51 | 0.22 |
| C | 2.3 | 600% | 1.14 | 1.53 | 0.45 |
| G | 3 | 30% | NA | NA | 0.05 |
| H | NA* | 610% | 1.04 | 1.22 | 0.24 |
| *NA indicates that the force is too small to be detected. | | | | | |

Method for measurement

Physical force: the scaffold material was cut into strips with a size of 10mm*60mm, infiltrated with water, and then the water at the surface was absorbed with a napkin. The rabbit skin in strip shape with a size of 10mm*60mm was taken. The two strips were intersectly overlapped at their both ends, slightly pressed to form a strip with a size of 10mm*70mm. When the relative movement between the overlapped strip and rabbit skin occurred, the maximal tensile force was measured by a tension testing machine.

Biological adhesive force: a white rabbit was anaesthetized with pentobarbital sodium, denuded; on the skin, a 5 cm*7 cm wound showing uniform dotted bleeding was created. The material with a size of 4cm*6cm was attached to the bleeding wound, compressed with a pressure of 40 mmHg for 5 min. A tension transducer (Type JN-IB-100) and a double channel physiological recorder (LMS-2B) were connected, then the material was dragged upward vertically with a constant velocity. The adhesive force was calculated based on the peak value of the maximal traction force.

Burst strength: the rabbit skin was taken and cut into circles with a diameter of 3 cm, then a hole with a diameter of 1 cm was cut at the center of the circle. The tissue repair scaffold of the present disclosure was taken and cut into a circular patch with a diameter of 3 cm, infiltrated and attached to the surface of rabbit skin, to ensure that the surface of the hole was completely covered by the tissue repair scaffold. Then the rabbit skin, onto which the scaffold was attached, was used to seal the opening of the container in which a pressure sensor was installed, to ensure the tight seal of the container. The air was evenly blown into the container, and when the tissue repair scaffold was forced open, the reading of the pressure sensor was recorded as the burst strength.

<Animal experiment

Experimental Comparative Example 1 (animal experiment)

Dogs were used in the animal experiment. The material prepared in Comparative Example 1 was used to perform the surgery of defect and repair of brain dura mater.

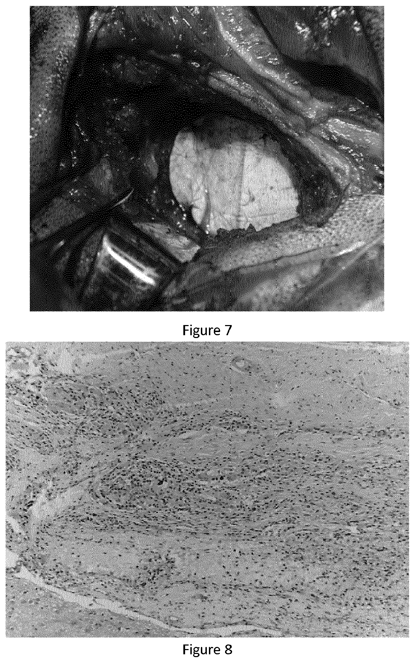

Animal experiment: six normal domestic dogs, with body weight of 15-20 kg, aged 1.5-2 years, were generally anesthetized via intramuscular injection of ketamine. Once anesthetized and denuded, the animal was placed on the operation table. The skin preparation area on the head was sterilized with 2% iodine tincture and 75% ethanol. The skin and the muscle beneath the skin were longitudinally incised along the midline of the head to expose the skull. The periosteum was separated with a detacher to expose the bone lamella of unilateral skull roof. The skull was ground with a high-speed drill, and a bone window was formed at unilateral skull roof. A piece of brain dura mater in elliptic shape with a size of 3cm*3cm was cut off from unilateral skull roof with ophthalmic scissors, and defect of brain dura mater was made at the roof part. The exposed surface of the brain was electrically burned, and six 1*1 mm defect dots were made. The material D was trimmed into repair material in the corresponding shape and size. The defects of brain dura mater of the experimental dogs were repaired through such methods: suture of brain dura mater was performed on 3 randomly selected dogs, while direct attachment to the brain dura mater was performed on the other 3 dogs. The attachment of the surgical material to the brain pia mater and brain cortex, in the suture surgery and non-suture surgery, was shown in Figure 7. Once the surgery was completed, the muscle and scalp skin were sutured with 4-0 silk suture thread.

Postoperative observation of the experimental dogs after the suture surgery: routine postoperative care and observation were performed on the animals. After the surgery, the animal recovered well. The surgical region healed well, and there was neither leakage of cerebrospinal fluid nor occurrence of epilepsy. The ingestion of food and water was normal, the outdoor activities of the animal were normal; no dyskinesia was observed. The animals survived throughout the observation period.

Eighteen months after the surgery, the sample and the tissue around were incised from the animal in such a way that, with the surgical site as the center, an area with an edge more than 1 cm from the center was incised, so that it included artificial brain dura mater, the brain dura mater around, and the brain tissue beneath the brain dura mater. It can be observed from the collected sample and tissue around that, the occlusion at the connection site between the material and the brain dura mater was smooth, there was no boundary, and the connection was completely sealed. The thickening of the brain dura mater and the fibrous encapsulation were not observed.

Observation of the experimental dogs that received non-suture surgery: routine postoperative care and observation were performed on the animals. After the surgery, the animals recovered slowly. There was subcutaneous effusion at the surgical site. The animals showed symptoms of epilepsy and dyskinesia. One of the animals died 1 day after the surgery, while the other two animals died on the 2nd day after the surgery. Leakage of cerebrospinal fluid and hemorrhagic effusion were observed from the incised wound.

Experimental Example 1 (animal experiment)

Dogs were used in the animal experiment. The material prepared in Example 1 was used to perform the surgery of defect and repair of brain dura mater.

Animal experiment: three normal domestic dogs, with body weight of 15-20 kg, aged 1.5-2 years, were generally anesthetized via intramuscular injection of ketamine. Once anesthetized and denuded, the animal was placed on the operation table. The skin preparation area on the head was sterilized with 2% iodine tincture and 75% ethanol. The skin and the muscle beneath the skin were longitudinally incised along the midline of the head to expose the skull. The periosteum was separated with a detacher to expose the bone lamella of unilateral skull roof. The skull was ground with a high-speed drill, and a bone window was formed at unilateral skull roof. A piece of brain dura mater in elliptic shape with a size of 3cm*3cm was cut off from unilateral skull roof with ophthalmic scissors, and defect of brain dura mater was made at the roof part. The exposed surface of the brain was electrically burned, and six 1*1 mm defect dots were made. The artificial brain dura matter prepared in Example 1 of the disclosure was trimmed into repair material in the corresponding shape and size. The defects at the roof of the dogs were repaired with the material in a manner of direct attachment. The material from Example 1 formed good attachment to the tissue (see Figure 6), which was in sharp contrast to the attachment status observed from the implantation of conventional materials (see Figure 7). Once the surgery was completed, the muscle and skin were sutured with suture thread.

Routine postoperative care and observation were performed on the animals. After the surgery, the animal recovered well. The surgical region healed well, and there was no subcutaneous effusion or leakage of cerebrospinal fluid, and no occurrence of epilepsy either. The ingestion of food and water, and mental condition of the animals were fine, and the motor function of the limbs was normal. The animals survived throughout the experimental period.

Eighteen months after the surgery, the sample was incised from the animal in such a way that, with the surgical site as the center, an area with an edge more than 1 cm from the center was incised, so that it included artificial brain dura mater, the brain dura mater around, and the brain tissue beneath the brain dura mater. It can be observed from the collected sample and tissue around that, the occlusion at the connection site between the material and the brain dura mater was smooth, there was no boundary, and the connection was completely sealed. From Figure 8 (pathological image) it can be observed that, at the surface of both sides of the implant, there were growth of a large amount of fibroblasts and deposition of collagen, fibers were regularly arranged, wherein filtration of a small number of lymphocytes was observed. The formation of a few capillaries was observed from the newly generated fibrous tissue. Abnormal reactions, such as hyperraemia, haemorrhage were not observed from the autologous brain dura mater.

Experimental Example 2 (animal experiment)

Dogs were used in the animal experiment. The material prepared in Example 7 was used to perform the surgery of brain dura mater repair.

Animal experiment: three normal domestic dogs, with body weight of 15-20 kg, aged 1.5-2 years, were generally anesthetized via intramuscular injection of ketamine. Once anesthetized and denuded, the animal was placed on the operation table. The skin preparation area on the head was sterilized with 2% iodine tincture and 75% ethanol. The skin and the muscle beneath the skin were longitudinally incised along the midline of the head to expose the skull. The periosteum was separated with a detacher to expose the bone lamella of unilateral skull roof. The skull was ground with a high-speed drill, and a bone window was formed at unilateral skull roof. A piece of brain dura mater in elliptic shape with a size of 3cm*3cm was cut off from unilateral skull roof with ophthalmic scissors, and defect of brain dura mater was made at the roof part. The exposed surface of the brain was electrically burned, and six 1*1 mm defect dots were made. The artificial brain dura matter prepared in Example 7 of the disclosure was trimmed into repair material in the corresponding shape and size. The material was used to repair the defects at the brain dura mater of the dog in a non-suture surgical method. Once the surgery was completed, the muscle and skin were sutured with suture thread.

Routine postoperative care and observation were performed on the animals. After the surgery, the animal recovered well. The surgical region healed well, and there was neither leakage of cerebrospinal fluid, nor occurrence of epilepsy. The ingestion of food and water was normal, the outdoor activities of the animal were normal; no dyskinesia was observed. The animals survived throughout the observation period.

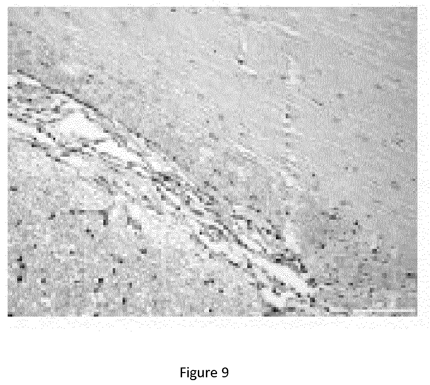

Eighteen months after the surgery, the sample was incised from the animal in such a way that, with the surgical site as the center, an area with an edge more than 1 cm from the center was incised, so that it included artificial brain dura mater, the brain dura mater around, and the brain tissue beneath the brain dura mater. It can be observed from the collected sample and tissue around that, the occlusion at the connection site between the material and the brain dura mater was smooth, there was no boundary, and the connection was completely sealed. From Figure 9 (pathological image) it can be observed that, at the surface of both sides of the implant, there were growth of a large amount of fibroblasts and deposition of collagen, fibers were regularly arranged, wherein filtration of a small number of lymphocytes was observed. The formation of a few capillaries was observed from the newly generated fibrous tissue. Abnormal reactions, such as hyperraemia, haemorrhage were not observed from the autologous brain dura mater.

Experimental Example 3 ((animal experiment)

The composite fiber membrane prepared in Example 4 and the membrane F prepared in Comparative Example 1 were cut into a size of 5cm*5cm, cleaned and sterilized, and used as artificial skin in the animal experiment. The rabbits used in the experiment had a body weight of 2-2.5 kg, aged 6-8 months, male or female, and the total number of the rabbits was 12. The rabbits were randomly assigned into 3 groups (experiment group, control group, blank group), with 4 rabbits in each group. The rabbits were generally anesthetized with injection through the vein of ear margin, and placed on a special operating table. The skin at the back was prepared, and routine preoperative sterilization was performed. The skin with a size of 4*4cm was incised from the back with a scalpel to make a defect of whole skin. The defect was repaired with artificial skin: in the experiment group, the artificial skin prepared from the composite fiber membrane of Example 4 was attached to the wound at the trauma site, pressed for 2 min. The membrane attached to the wound. In the control group, the artificial skin prepared from membrane F was attached onto the wound site. The material could not adhere to the wound, in contrast, it had to be fixed with suture thread every 1 cm. In the blank control group, the wound was not treated with artificial skin. In experiment group, control group and blank group, the wounds were respectively covered with a layer of sterile oiled yarn and gauze, which were fixed to the skin around with suture thread.

After the surgery, the animals were observed for ingestion of food and water, body temperature, the time period that the cover material detached from the wound, and other physiological activities. The healing of the wound was also observed. At the 4th and 8th week after the surgery, 2 rabbit from each group were taken and euthanized. The whole wound on the back and the normal skins around the wound were taken, fixed with formalin, stained by HE staining method. The newly generated tissue in the dermis, the inflammation reaction, the thickness of the epidermis structure, and the regeneration of the skin appendages were observed with optical microscopy.

After the surgery, the rabbits in blank group have all the wounds stuck to the oiled yarn or gauze. There was a phenomenon that the dressing detached from the wound. There was bleeding at the wound, and exudation of a large amount of tissue fluid. After complete healing, the wound showed linear scar.

In the control group, the artificial skin at the wound was not smooth, while the edge of the artificial skin did not attach to the wound well. There was slight redness, swelling, heat, and slight inflammation at the wound, and a few exudation of tissue fluid. There was slight sticking between the wound and the gauze. After complete healing, the wound showed a few scars in rectangular shape and a lot of linear scars.

In the experiment group, the wound was dry after the surgery, and closely attached to the artificial skin, without sticking to the oiled yarn and gauze. There was no redness, swelling or heat at the wound. The animals did not show phenomenon like skin allergy or infection. There was no necrosis at the part covered by the material. After complete healing, the wound showed scars in rectangular shape. It was observed through microscopy that the prickle cell layer of the healed skin thickened, the cells in stratum layer proliferated actively; the keratin layer, granular layer, prickle cell layer and the basal layer of the epidermis were all observable. In the dermis layer, the appendages, and the structure of sweat glands and hair follicles were observable. It could be observed that a lot of fibroblasts arranged in parallel with the surface of the skin, in which there was relatively large amount of capillaries in proliferation, and topical infiltration of lymphocytes.

The result of the observation shows that, the attachment, anti-infection property and the function of wound bleeding prevention of the material in experiment group were better than those in the control group and blank group. Redness, swelling or necrosis was not seen from the wound in experiment group. The histological observation shows that, the artificial skin prepared from the composite fiber membrane in Example 4 has a wound attachment capability better than that in control group and blank group, and can more efficiently promote the regeneration of skin structure.

Experimental Example 4 (animal experiment)

The scaffold B prepared in Example 11 and the scaffold G prepared in Comparative Example 2 were respectively cut into a size of 5cm*5cm, cleaned and sterilized, and used as artificial skin in the animal experiment. The rabbits used in the experiment had a body weight of 2-2.5 kg, aged 6-8 months, male or female, and the total number of the rabbits was 12. The rabbits were randomly assigned into 3 groups (experiment group, control group, blank group), with 4 rabbits in each group. The rabbits were generally anesthetized with injection through the vein of ear margin, and placed on a special operating table. The skin at the back was prepared, and routine preoperative sterilization was performed. The skin with a size of 4*4cm was incised from the back with a scalpel to make a defect of whole skin. The defect was repaired with artificial skin: in the experiment group, the artificial skin prepared from the scaffold B was attached to the wound at the trauma site, pressed for 2 min. The membrane attached to the wound. In the control group, the artificial skin prepared from scaffold G was attached onto the wound site. The material could not adhere to the wound; in contrast, it had to be fixed with suture thread every 1 cm. In the blank control group, the wound was not treated with artificial skin. In experiment group, control group and blank group, the wounds were respectively covered with a layer of sterile oiled yarn and gauze, which were fixed to the skin around with suture thread.

After the surgery, the animals were observed for ingestion of food and water, body temperature, the time period that the cover material detached from the wound, and other physiological activities. The healing of the wound was also observed. At the 4th and 8th week after the surgery, 2 rabbit from each group were taken and euthanized. The whole wound on the back and the normal skins around the wound were taken, fixed with formalin, stained by HE staining method. The newly generated tissue in the dermis, the inflammation reaction, the thickness of the epidermis structure, and the regeneration of the skin appendages were observed with optical microscopy.

After the surgery, the rabbits in blank group have all the wounds stuck to the oiled yarn or gauze. There was a phenomenon that the dressing detached from the wound. There was bleeding at the wound, and exudation of a large amount of tissue fluid. After complete healing, the wound showed linear scar.

In the control group, the artificial skin at the wound was not smooth, the edge of the artificial skin was not attached to the wound, and there was a phenomenon that the artificial skin raised or detached from the wound. There was slight redness, swelling, heat, and slight infection at the wound, and a few exudation of tissue fluid. There was slight sticking between the wound and the gauze. After complete healing, the wound showed a few scars in rectangular shape and a lot of linear scars.

In the experiment group, the wound was dry after the surgery, and closely attached to the artificial skin, without sticking to the oiled yarn and gauze. There was no redness, swelling or heat at the wound. The animals did not show phenomenon like skin allergy or infection. There was no necrosis at the part covered by the material. After complete healing, the wound showed scars in rectangular shape. It was observed through microscopy that the prickle cell layer of the healed skin thickened, the cells in stratum layer proliferated actively; the keratin layer, granular layer, prickle cell layer and the basal layer of the epidermis were all observable. In the dermis layer, the appendages, and the structure of sweat glands and hair follicles were observable. It could be observed that a lot of fibroblasts arranged in parallel with the surface of the skin, in which there was relatively large amount of capillaries in proliferation, and topical infiltration of lymphocytes.

The result of the observation shows that, the attachment, anti-infection property and the function of wound bleeding prevention of the material in experiment group were better than those in the control group and blank group. Redness, swelling or necrosis was not seen from the wound in experiment group. The histological observation shows that, the artificial skin prepared from the scaffold B of Example 11 has a wound attachment capability which is absent in control group and blank group, and can more efficiently promote the regeneration of skin structure.

The description above is only the specific embodiments of the present disclosure, and the protection scope of the present disclosure is not limited to it. In the technical scope of the present disclosure, the variation or replacement which can be easily conceived by anyone skilled in the art should be encompassed within the scope of the disclosure. Therefore, the protection scope of the present application should be defined by the claims appended thereto.

Applicability

The tissue repair scaffolds provided in the Examples according to the present disclosure can be used in the technical field of medical device, and are particularly suitable for use as brain dura matter repairing material, spinal dura matter repairing material, a tissue engineering scaffold material, artificial skin, a wound dressing, a biological membrane, a wound cladding material, a hemostatic material, a postoperative adhesion prevention material, or a plastic and aesthetic material.

**Claims**

1. A tissue repair scaffold, wherein the tissue repair scaffold comprises a surface of at least one layer of composite fiber layer, said composite fiber layer comprises composite fibers formed with an adhesion factor and a hydrophobic synthetic material, said composite fiber layer being formed by interweaving composite fiberfilaments with diameters of 10 nm-100 $\mu$m, and the content of said adhesion factor being 5%-50% by mass based on the total weight of the composite fiber.

2. The tissue repair scaffold according to claim 1, wherein the tissue repair scaffold comprises a porous three-dimensional reticulate structure formed with the composite fiber layer and a hydrophilic material; and said porous three-dimensional reticulate structure being formed on and/or inside a surface of the composite fiber layer.

3. The tissue repair scaffold according to claim 2, wherein the hydrophilic material is selected from a group consisting of hydrophilic proteins, celluloses, alcohols, ethers, chitosans, saccharides, nitrogen-containing hydrophilic substances.

4. The tissue repair scaffold according to claim 2 or 3, wherein the porous three-dimensional reticulate structure is formed through the steps of: immersing the composite fiber layer into a solution of hydrophilic material; taking out the composite fiber layer; and freeze-drying the composite fiber layer, said solution of hydrophilic material preferably being an aqueous solution of water-soluble polymer.

5. The tissue repair scaffold according to any one of claim 2-4, wherein when the tissue repair scaffold is immersed in water for 10s, a water absorption ratio thereof may reach 100-5000%.

6. The tissue repair scaffold according to any one of claims 2-5, wherein:

   - the tensile strength of the tissue repair scaffold is larger than 1 Mpa; and/or
   - once the tissue repair scaffold attaches to a tissue around, a burst strength is 0.10 kPa or more; and/or
   - a pore size of the tissue repair scaffold is 1.3-500 $\mu$m.

7. The tissue repair scaffold according to any one of claims 1-6, wherein a hydrophilic-lipophilic balance (HLB) value of the tissue repair scaffold is 1-13.

8. The tissue repair scaffold according to any one of claims 1-7, wherein a contact angle $\theta$ between the surface of the tissue repair scaffold and water is 90° or less.

9. The tissue repair scaffold according to any one of claims 1-8, wherein:

   - the adhesion factor which forms the composite fiber may be present inside the composite fiber and/or at the surface of the composite fiber; and/or
   - the adhesion factor preferably being one or more selected from proteins with hydrophilicity and derivative materials thereof, celluloses with hydrophilicity and derivative materials thereof, alcohols with hydrophilicity and derivative materials thereof, chitosans and derivative materials thereof, saccharides and derivative materials thereof, and nitrogen-containing hydrophilic substances; and/or
   - the content of said adhesion factor is 10%-50% by mass, preferably 15%-30% by mass, based on a total weight of the composite fiber.

10. The tissue repair scaffold according to any one of claims 1-9, wherein the hydrophobic synthetic material includes hydrophobic polyurethane, polyethylene terephthalate, polycaprolactone, polyglycolic acid, poly(lactic acid-glycolic acid) copolymers, poly(lactic acid-caprolactone) copolymers, or polylactic acid.

11. The tissue repair scaffold according to any one of claims 1-10, wherein:

    - the composite fiber layer has a porous structure; and/or
    - the composite fiber layer has a pore size of 1-100 $\mu$m.

12. The tissue repair scaffold according to any one of claims 1-11, for use as brain dura matter repairing material, spinal dura matter repairing material, a tissue engineering scaffold material, artificial skin, wound dressing, a biological membrane, a wound cladding material, a hemostatic material, a postoperative adhesion prevention material or a plastic and aesthetic material.

13. A preparation method for the tissue repair scaffold according to any one of claims 1-12, wherein the composite fiber layer of the tissue repair scaffold is prepared through steps of:
dissolving the adhesion factor and the hydrophobic synthetic material in the same kind of solvent; and then obtaining a membrane containing composite fibers by means of electrostatic spinning, centrifugal spinning, melt blowing, melt electrospinning or a combination thereof, said solvent used in the steps of preparing the composite fiber layer preferably at least comprising one kind of fluorine-containing solvent, said fluorine-containing solvent preferably including hexafluoroisopropanol, trifluoroethanol, or trifluoroacetic acid.

14. The preparation method according to claim 13, wherein said preparation method further comprises a step of preparing the porous 3D reticulate structure by immersing the prepared membrane containing composite fibers into a solution of hydrophilic material, taking out the membrane; and freeze-drying the membrane.

**Patentansprüche**

1. Gewebereparaturgerüst, wobei das Gewebereparaturgerüst eine Oberfläche von mindestens einer Schicht einer Verbundfaserschicht umfasst, wobei die Verbundfaserschicht Verbundfasern umfasst, die mit einem Adhäsionsfaktor und einem hydrophoben synthetischen Material gebildet sind, wobei die Verbundfaserschicht durch Verweben von Verbundfaserfilamenten mit Durchmessern von 10 nm - 100 $\mu$m gebildet sind und der Gehalt an Adhäsionsfaktor bezogen auf das Gesamtgewicht der Verbundfaser 5-50 Masseprozent beträgt.

2. Gewebereparaturgerüst nach Anspruch 1, wobei das Gewebereparaturgerüst eine poröse, dreidimensionale, netzartige Struktur umfasst, die mit der Verbundfaserschicht und einem hydrophilen Material gebildet ist; und die poröse, dreidimensionale, netzartige Struktur auf und/oder in einer Oberfläche der Verbundfaserschicht gebildet ist.

3. Gewebereparaturgerüst nach Anspruch 2, wobei das hydrophile Material ausgewählt ist aus einer Gruppe, bestehend aus hydrophilen Proteinen, Cellulosen, Alkoholen, Ethern, Chitosanen, Sacchariden, stickstoffhaltigen hydrophilen Substanzen.

4. Gewebereparaturgerüst nach Anspruch 2 oder 3, wobei die poröse, dreidimensionale, netzartige Struktur durch die folgenden Schritte gebildet wird: Eintauchen der Verbundfaserschicht in eine Lösung von hydrophilem Material; Herausnehmen der Verbundfaserschicht; und Gefriertrocknen der Verbundfaserschicht, wobei die Lösung von hydrophilem Material vorzugsweise eine wässrige Lösung von wasserlöslichem Polymer ist.

5. Gewebereparaturgerüst nach einem der Ansprüche 2-4, wobei, wenn das Gewebereparaturgerüst für 10 s in Wasser getaucht wird, ein Wasserabsorptionsverhältnis davon 100-5000 % erreichen kann.

6. Gewebereparaturgerüst nach einem der Ansprüche 2-5, wobei:

   - die Zugfestigkeit des Gewebereparaturgerüsts größer als 1 MPa ist; und/oder,
   - sobald sich das Gewebereparaturgerüst an ein umliegendes Gewebe heftet, eine Berstfestigkeit 0,10 kPa oder mehr beträgt; und/oder
   - eine Porengröße des Gewebereparaturgerüsts 1,3-500 $\mu$m beträgt.

7. Gewebereparaturgerüst nach einem der Ansprüche 1-6, wobei ein Hydrophil-Lipophil-Gleichgewichtswert (HLB-Wert) des Gewebereparaturgerüsts 1-13 beträgt.

8. Gewebereparaturgerüst nach einem der Ansprüche 1-7, wobei ein Kontaktwinkel $\theta$ zwischen der Oberfläche des Gewebereparaturgerüsts und Wasser 90° oder weniger beträgt.

9. Gewebereparaturgerüst nach einem der Ansprüche 1-8, wobei:

   - der Adhäsionsfaktor, der die Verbundfaser bildet, in der Verbundfaser und/oder an der Oberfläche der Verbundfaser vorhanden sein kann; und/oder
   - der Adhäsionsfaktor vorzugsweise einer oder mehrere, ausgewählt aus Proteinen mit Hydrophilie und Derivatmaterialien davon, Cellulosen mit Hydrophilie und Derivatmaterialien davon, Alkoholen mit Hydrophilie und Derivatmaterialien davon, Chitosanen und Derivatmaterialien davon, Sacchariden und Derivatmaterialien davon und stickstoffhaltigen hydrophilen Substanzen, ist; und/oder
- der Gehalt an dem Adhäsionsfaktor bezogen auf ein Gesamtgewicht der Verbundfaser 10-50 Masseprozent, vorzugsweise 15-30 Masseprozent, beträgt.

10. Gewebereparaturgerüst nach einem der Ansprüche 1-9, wobei das hydrophobe synthetische Material hydrophobes Polyurethan, Polyethylenterephthalat, Polycaprolacton, Polyglycolsäure, Poly(Milchsäure-Glycolsäure)-Copolymere, Poly(Milchsäure-Caprolacton)-Copolymere oder Polymilchsäure einschließt.

11. Gewebereparaturgerüst nach einem der Ansprüche 1-10, wobei:

- die Verbundfaserschicht eine poröse Struktur aufweist; und/oder
- die Verbundfaserschicht eine Porengröße von 1-100 $\mu$m aufweist.

12. Gewebereparaturgerüst nach einem der Ansprüche 1-11 zur Verwendung als Reparaturmaterial für zerebrale Dura mater, Reparaturmaterial für spinale Dura mater, Gerüstmaterial für Gewebezüchtung, künstliche Haut, Wundverband, eine biologische Membran, ein Wundabdeckmaterial, ein hämostatisches Material, ein Material zur Verhinderung postoperativer Adhäsion oder ein plastisches und ästhetisches Material.

13. Herstellungsverfahren für das Gewebereparaturgerüst nach einem der Ansprüche 1-12, wobei die Verbundfaserschicht des Gewebereparaturgerüsts durch folgende Schritte hergestellt wird:
Lösen des Adhäsionsfaktors und des hydrophoben synthetischen Materials in der gleichen Art von Lösungsmittel; und anschließendes Erhalten einer Verbundfasern enthaltenden Membran durch elektrostatisches Spinnen, zentrifugales Spinnen, Schmelzblasen, Schmelzelektrospinnen oder eine Kombination davon, wobei das in den Schritten der Herstellung der Verbundfaserschicht verwendete Lösungsmittel vorzugsweise mindestens eine Art von fluorhaltigem Lösungsmittel umfasst, wobei das fluorhaltige Lösungsmittel vorzugsweise Hexafluorisopropanol, Trifluorethanol oder Trifluoressigsäure einschließt.

14. Herstellungsverfahren nach Anspruch 13, wobei das Herstellungsverfahren weiter einen Schritt des Herstellens der porösen, netzförmigen 3D-Stuktur durch Eintauchen der Verbundfasern enthaltenden hergestellten Membran in eine Lösung von hydrophilem Material, Herausnehmen der Membran; und Gefriertrocknen der Membran umfasst.

**Revendications**

1. Support de réparation de tissu, dans lequel le support de réparation de tissu comprend une surface d'au moins une couche de couche de fibres composites, ladite couche de fibres composites comprend des fibres composites formées avec un facteur d'adhésion et un matériau synthétique hydrophobe, ladite couche de fibres composites étant formée par l'entrelacement de filaments de fibres composites ayant des diamètres allant de 10 nm à 100 $\mu$m et la teneur dudit facteur d'adhésion étant comprise entre 5% et 50% en masse par rapport au poids total de la fibre composite.

2. Support de réparation de tissu selon la revendication 1, dans lequel le support de réparation de tissu comprend une structure réticulée tridimensionnelle poreuse formée avec la couche de fibres composites et un matériau hydrophile ; et ladite structure réticulée tridimensionnelle poreuse étant formée sur et/ou à l'intérieur d'une surface de la couche de fibres composites.

3. Support de réparation de tissu selon la revendication 2, dans lequel le matériau hydrophile est choisi dans un groupe constitué de protéines hydrophiles, de celluloses, d'alcools, d'éthers, de chitosanes, de saccharides, de substances hydrophiles contenant de l'azote.

4. Support de réparation de tissu selon la revendication 2 ou 3, dans lequel la structure réticulée tridimensionnelle poreuse est formée par les étapes consistant : à immerger la couche de fibres composites dans une solution de matériau hydrophile ; à retirer la couche de fibres composites ; et à lyophiliser la couche de fibres composites, ladite solution de matériau hydrophile étant de préférence une solution aqueuse de polymère hydrosoluble.

5. Support de réparation de tissu selon l'une quelconque des revendications 2 à 4, dans lequel, lorsque le support de réparation de tissu est immergé dans l'eau pendant 10 secondes, un rapport d'absorption d'eau de celui-ci peut atteindre 100 à 5000%.

6. Support de réparation de tissu selon l'une quelconque des revendications 2 à 5, dans lequel :

   - la résistance à la traction du support de réparation de tissu est supérieure à 1 MPa ; et/ou
   - une fois que le support de réparation de tissu est fixé à un tissu environnant, la résistance à l'éclatement est supérieure ou égale à 0,10 kPa ; et/ou
   - la taille de pores du support de réparation de tissu est comprise entre 1,3 et 500 $\mu$m.

7. Support de réparation de tissu selon l'une quelconque des revendications 1 à 6, dans lequel une valeur d'équilibre hydrophile-lipophile (HLB) du support de réparation de tissu est comprise entre 1 et 13.

8. Support de réparation de tissu selon l'une quelconque des revendications 1 à 7, dans lequel un angle de contact $\theta$ entre la surface du support de réparation de tissu et l'eau est inférieur ou égal à 90°.

9. Support de réparation de tissu selon l'une quelconque des revendications 1 à 8, dans lequel :

   - le facteur d'adhésion qui forme la fibre composite peut être présent à l'intérieur de la fibre composite et/ou à la surface de la fibre composite ; et/ou
   - le facteur d'adhésion étant de préférence un ou plusieurs facteur(s) choisi(s) parmi des protéines à caractère hydrophile et des matériaux dérivés de celles-ci, des celluloses à caractère hydrophile et des matériaux dérivés de celles-ci, des alcools à caractère hydrophile et des matériaux dérivés de ceux-ci, des chitosanes et des matériaux dérivés de ceux-ci, des saccharides et des matériaux dérivés de ceux-ci, et des substances hydrophiles contenant de l'azote ; et/ou
   - la teneur dudit facteur d'adhésion est comprise entre 10% et 50% en masse, de préférence entre 15% et 30% en masse, par rapport au poids total de la fibre composite.

10. Support de réparation de tissu selon l'une quelconque des revendications 1 à 9, dans lequel le matériau synthétique hydrophobe comporte du polyuréthane hydrophobe, du polyéthylène téréphtalate, de la polycaprolactone, de l'acide polyglycolique, des copolymères poly(acide lactique-acide glycolique), des copolymères poly(acide lactique-caprolactone), ou de l'acide polylactique.

11. Support de réparation de tissu selon l'une quelconque des revendications 1 à 10, dans lequel :

   - la couche de fibres composites a une structure poreuse ; et/ou
   - la couche de fibres composites a une taille de pores allant de 1 à 100 $\mu$m.

12. Support de réparation de tissu selon l'une quelconque des revendications 1 à 11, pour une utilisation comme un matériau de réparation de la dure-mère du cerveau, un matériau de réparation de la dure-mère spinale, un matériau de support d'ingénierie tissulaire, une peau artificielle, un pansement, une membrane biologique, un matériau de revêtement de plaie, un matériau hémostatique, un matériau anti-adhésion postopératoire ou une matière plastique et esthétique.

13. Procédé de préparation du support de réparation de tissu selon l'une quelconque des revendications 1 à 12, dans lequel la couche de fibres composites du support de réparation de tissu est préparée par les étapes consistant : à dissoudre le facteur d'adhésion et le matériau synthétique hydrophobe dans le même type de solvant ; et à obtenir ensuite une membrane contenant des fibres composites au moyen d'un filage électrostatique, d'un filage centrifuge, d'un procédé de fusion-soufflage, d'un électrofilage par fusion ou d'une combinaison de ceux-ci, ledit solvant étant utilisé dans les étapes de préparation de la couche de fibres composites, de préférence comprenant au moins un type de solvant contenant du fluor, ledit solvant contenant du fluor comportant de préférence de l'hexafluoroisopropanol, du trifluoroéthanol ou de l'acide trifluoroacétique.

14. Procédé de préparation selon la revendication 13, dans lequel ledit procédé de préparation comprend en outre une étape consistant à préparer la structure réticulée 3D poreuse par immersion de la membrane préparée contenant des fibres composites dans une solution de matériau hydrophile, retrait de la membrane ; et lyophilisation de la membrane.

（A）    （B）

Figure 1

Figure 2

Figure 3

Figure 4

| 20 µm | Mag = 200 X | EHT = 3.00 kV | | Date :27 Nov 2013 | ZEISS |
| | WD = 6.3 mm | Signal A = SE2 | JNU | Time :14:11:55 | |

Figure 5

Figure 6

Figure 7

Figure 8

Figure 9